# FASCICULE DE BREVET EUROPEEN

(11) **EP 3 104 971 B1**
(45) Date de publication et mention de la délivrance du brevet: **28.08.2019**
(21) Numéro de dépôt: 15706864.4
(22) Date de dépôt: 27.01.2015
(51) Int. Cl.: B01J 37/03, A61L 9/20, B01J 21/06, B01J 35/00, B01J 35/02, B01J 35/10, C01B 3/04, C02F 1/32

(54) **MATÉRIAU À BASE DE TIO2 ABSORBANT DANS LE VISIBLE ET PROCÉDÉ POR SA FRABRICATION**
TIO2-BESTEHENDES PRODUKT, DAS SICHTBARES LICHT ABSORBIERT, UND DESSEN HERSTELLUNGSVERFAHREN
TIO2-BASED MATERIAL ABSORBING VISIBLE LIGHT AND ITS MANUFACTURE PROCESS

(30) Priorité: 27.01.2014 FR 1400194
(43) Date de publication de la demande: 21.12.2016
(73) Titulaire: Total S.A., 92400 Courbevoie (FR); Centre National de la Recherche Scientifique (C.N.R.S.), 75016 Paris (FR); Ecole Nationale Supérieure de Chimie de Montpellier, 34090 Montpellier (FR)
(72) Inventeur: BOSCARO, Paolo, F-75016 Paris (FR); HULEA, Vasile, F-34090 Montpellier (FR); MARCOTTE, Nathalie, F-34090 Montpellier (FR); FAJULA, François, F-34820 Teyran (FR); GALARNEAU, Anne, F-34090 Montpellier (FR); LUCK, Francis, F-93160 Noisy le Grand (FR)
(74) Mandataire: Cabinet Plasseraud
(86) Numéro de dépôt international: PCT/FR2015/050187
(87) Numéro de publication internationale: WO 2015/110772

(56) Documents cités:
- CN-A- 101 462 068
- CN-A- 101 721 985
- CN-A- 102 451 671
- US-A1- 2011 028 311
- US-A1- 2012 118 723
- US-A1- 2012 165 184
- SENTHILNATHAN J ET AL: "Photodegradation of methyl parathion and dichlorvos from drinking water with N-doped TiOunder solar radiation", CHEMICAL ENGINEERING JOURNAL, ELSEVIER SEQUOIA, LAUSANNE, CH, vol. 172, no. 2, 15 juin 2011 (2011-06-15) , pages 678-688, XP028270639, ISSN: 1385-8947, DOI: 10.1016/J.CEJ.2011.06.035 [extrait le 2011-06-22] cité dans la demande

## Description

### Domaine technique

Le domaine technique dans lequel se situe particulièrement l'invention est celui des matériaux utilisables en tant que photocatalyseurs activés par rayonnement dans le spectre visible, et plus particulièrement celui des matériaux à base d'oxyde de titane (TiO₂) utilisables en tant que photocatalyseurs activés par rayonnement dans le spectre visible, notamment pour la dégradation de polluants dans l'air et dans l'eau ou le craquage de l'eau, et leurs procédés de fabrication.

### État de la technique

L'état de la technique montre actuellement que l'obtention d'un taux de conversion (c'est-à-dire le rapport entre la quantité de réactif transformé et la quantité totale de réactif présent dans un volume donné et à un temps donné) élevé nécessite le développement de matériaux nanostructurés fonctionnant comme catalyseur avec une composition bien définie et contrôlée ainsi qu'une forme spécifique.

En ce qui concerne les photocatalyseurs à base de TiO₂, parmi les meilleurs résultats obtenus on peut citer des catalyseurs comprenant des nanotubes de TiO₂ que l'on fait croître sur des feuilles en titane. Ces photocatalyseurs sont activés par rayonnement exclusivement dans le spectre visible.

Bien que ces photocatalyseurs soient très efficaces en phase gazeuse, leur utilisation en phase liquide ne peut être envisagée à cause de l'instabilité des nanotubes déposés sur les feuilles.

D'autres photocatalyseurs à base de TiO₂ ont été étudiés. Notamment, le TiO₂ Degussa P25 fait figure de photocatalyseur à base de TiO₂ de référence. Ce photocatalyseur s'avère être efficace sous rayonnement UV. Cependant, sa capacité à absorber la lumière n'est pas satisfaisant.

Afin d'améliorer la capacité des photocatalyseurs à base de TiO₂ à absorber la lumière, des particules de métaux nobles, tels que le platine, le palladium, l'or et l'argent, ont été ajoutées.

Le document « Influence de la longueur d'onde d'excitation (UV ou lumière visible) sur l'activité photocatalytique de l'oxyde de titane comprenant des nanoparticules d'or pour la génération d'hydrogène ou d'oxygène à partir d'eau » (titre original en anglais : « Influence of Excitation Wavelength (UV or Visible Light) on the Photocatalytic Activity of Titania Containing Gold Nanoparticles for the Generation of Hydrogen or Oxygen from Water », G.C. Silva et al., J. Am. Chem. Soc., 2011, 113, pp 595-602) et « Photocatalyseur plasmonique pour l'évolution de l'hydrogène dans le craquage catalytique de l'eau » (titre original en anglais : « Plasmonic Photocatalyst for H2 Evolution in Photocatalytic Water Splitting », J.J. Chen et al., J. Phys. Chem. C, 2011, pages 115-210) décrivent des procédés pour la synthèse de photocatalyseurs mésoporeux à base de TiO₂ et d'or pour le craquage de l'eau. Ces matériaux sont nanométriques et sont constitués de particules de TiO₂ « P25 » de 20 nm à 30 nm de diamètre avec des particules d'or de 2 nm à 4 nm de diamètre.

L'absorption de ces matériaux est intense dans l'UV, notamment à 300 nm, mais est nulle à 400 nm et extrêmement faible entre 500 nm et 600 nm.

Le document « Amélioration de l'activité photocatalytique par déposition de métal : caractéristation et efficacité photonique de Pt, Au et Pd déposé sur un catalyseur à base de TiO2 » (titre original en anglais : « Enhancement of photocatalytic activity by metal deposition: characterisation and photonic efficiency of Pt, Au and Pd depositied on TiO2 catalyst », S. Sakthivel et al., Water Research, 2004, 38, pages 3001-3008) décrit un procédé pour la synthèse d'un photocatalyseur à base de TiO₂ présentant du platine, de l'or ou du palladium déposé en surface pour la dégradation de colorants organiques, notamment l'acide green 16 sous rayonnement UV. Ce matériau présente une absorption à 500 nm supérieure à celle à 400 nm.

Les articles « Préparation et caractérisation des photocatalyseurs à base de titane mésoporeux, comprenant de l'argent de taille nanométrique pour la dégradation d'herbicide » (titre original en anglais : « Préparation and characterisation of nano-silver/mesoporous titania photocatalysts for herbicide degradation », M.M. Mohamed et al., Microporous and Mesoporous Materials, 142 (2011), pages 130 à 138) et « Dégradation photocatalytique d'herbicides sélectionnés dans des suspensions aqueux de TiO2 dopé sous rayonnement visible » (titre original en anglais : « Photocatalytic degradation of selected herbicides in aqueous suspensions of doped titania under visible light irradiation », D. V. Sojic et al., J. Harzardous Materials, 179 (2010), pages 49 à 56) décrivent la synthèse de matériaux à base de TiO₂ pour la dégradation d'herbicides dans l'eau. Ces matériaux comprennent par ailleurs également de l'argent, du fer ou de l'azote. Le matériau à base de TiO₂ et d'argent est synthétisé à partir d'éther de polyoxyéthylène lauryle (POL), d'AgNO₃ et de Ti(OiPr)₄ et se présente sous forme de nanoparticules de diamètre compris entre 15 et 25 nm. L'absorption à la lumière visible n'est pas donnée pour ce matériau. Le matériau à base de TiO₂ et de Fe est synthétisé à partir de FeCl₃ (trichlorure de fer III) et de TiCl₄ (tétrachlorure de titane). Il se présente sous la forme de nanoparticules de diamètre compris entre 5 et 7 nm. La réflectance de ce matériau est quasiment de 100% à 500 nm correspondant à une absorbance F(R) (Kubelka-Munk) de 0. Le matériau à base de TiO₂ et d'azote est synthétisé à partir de Ti(OH)₄ (tétrahydroxyde de titane) et d'urée. Il se présente sous la forme de nanoparticules de diamètre compris entre 6 et 8 nm et sa réflectance à 400 nm est proche de 40% correspondant à une absorbance F(R) (Kubelka-Munk) de 0.44 alors que celle à 500 nm est proche de 90% correspondant à une absorbance F(R) (Kubelka-Munk) de 0,005, soit une absorption de la lumière à 500 nm de 1 % de l'absorption de la lumière à 400 nm.

L'article « Dégradation photocatalytique de lindane sous lumière UV et visible utilisant du TiO2 dopé au N » (titre original en anglais : « Photocatalytic degradation of lindane under UV and visible light using N-doped TiO2 », J. Senthilnathan et al., Chem. Eng. J., 161 (2010), pages 83 à 92) décrit la synthèse d'un matériau à base de TiO₂ dopé à l'azote pour la dégradation d'un pesticide. Ce matériau est obtenu à partir Ti(OiPr)₄ dans EtOH et N(CH₃CH₂)₃ (triéthylamine) en milieu acide. Il se présente sous forme de nanoparticules de taille comprise entre 29 et 71 nm.

Le document CN102744059 décrit un procédé pour la synthèse d'un photocatalyseur mésoporeux en TiO₂ et en argent pour la dépollution de l'eau de lac, de mer, des égouts et des eaux usées industrielles.

Le document CN10245167 décrit un matériau à base de TiO₂ dopé à l'azote, la teneur de l'azote étant de 0,03 à 0,05 at% et l'oxyde de titane sous forme d'anatase. Le matériau présente des macropores de 200 à 300 µm, des mésopores de 2 à 4 nm, des micropores de 1 à 2 nm et une porosité de 80 à 90 %.

Ainsi, certains de ces photocatalyseurs nécessitent l'utilisation de nanoparticules de métaux nobles et sont par conséquent plus onéreux.

Par ailleurs, la plupart de ces photocatalyseurs peuvent être difficiles à synthétiser. En outre, ils sont sujets au mûrissement d'Ostwald, phénomène dans lequel les nanoparticules s'agrègent les unes aux autres ce qui provoque leur désactivation progressive, par conséquent, les nanoparticules sont peu stables thermiquement. Par ailleurs, les nanoparticules à base de TiO₂ en suspension dans une solution sont difficiles à séparer de cette solution.

Ainsi, un matériau à base de TiO₂ présentant une absorbance dans le spectre visible, dont la fabrication est peu onéreuse et dont la manipulation est aisée constitue toujours un besoin qui n'est à ce jour pas encore satisfait.

### Présentation de l'invention

Un objectif est donc ici notamment de proposer un procédé permettant la fabrication d'un matériau qui pallie les inconvénients de l'art antérieur présenté ci-dessus.

Pour cela, un procédé de production d'un matériau à base de TiO2 est proposé. Ce procédé comprend :
- le mélange préalable d'un alcoxyde de titane avec un acide ;
- l'ajout d'eau, d'un séparateur de phase et d'une source de N au mélange d'alcoxyde de titane et d'acide pour obtenir un gel de TiO2 ;
- le lavage du gel de TiO2 avec de l'isopropanol (iPrOH) ;
- le séchage et la calcination du gel de TiO₂ afin de produire le matériau à base de TiO₂ exempt de métaux et présentant des mésopores et macropores ouvertes, les mésopores présentant un diamètre compris entre 2 nm et 10 nm, les macropores présentant un diamètre compris entre 2 µm et 10 µm, le volume mésoporeux étant supérieur à 0,1 cm³/g et le volume macroporeux étant supérieur à 0,3 cm³/g, le matériau à base de TiO₂ comprenant au moins 60 %, de préférence au moins 70 %, en poids de TiO₂ sous forme d'anatase et du carbone élémentaire et/ou de l'azote élémentaire, le carbone élémentaire et/ou l'azote élémentaire étant chacun en une quantité inférieure ou égale à environ 2 % en poids, les pourcentages étant calculés par rapport au poids total du matériau.

Un tel procédé a pour avantage non négligeable de permettre la fabrication d'un matériau à base de TiO₂ présentant une absorbance dans le spectre visible de sorte que l'absorption de la lumière à 500 nm soit supérieure à 50 % de l'absorption de la lumière à 400 nm.

L'alcoxyde de titane est de préférence Ti(OiPr)₄.

Le ratio molaire alcoxyde de titane:acide est de préférence compris entre 1:1 et 1:0,5.

L'acide peut être du HCl.

Le séparateur de phase est avantageusement du poly(oxyde d'éthylène) (PEO), de préférence à une masse molaire moyenne en nombre (Mn) supérieure ou égale à 10 000.

Le ratio molaire alcoxyde de titane:source de N peut être compris entre 1:1 et 1:0,75.

La source de N est de préférence du N-méthyleformamide (NFA).

Il est également proposé un matériau à base de TiO₂ et exempt de métaux ainsi que des additifs autres que C et N, le matériau présentant des mésopores et macropores ouvertes, les mésopores présentant un diamètre compris entre 2 nm et 10 nm, les macropores présentant un diamètre compris entre 2 µm et 10 µm, le volume mésoporeux étant supérieur à 0,1 cm³/g et le volume macroporeux étant supérieur à 0,3 cm³/g, le matériau à base de TiO₂ comprenant au moins 60 %, de préférence au moins 70 %, en poids de TiO₂ sous forme d'anatase et du carbone élémentaire et/ou de l'azote élémentaire, le carbone élémentaire et/ou l'azote élémentaire étant chacun en une quantité inférieure ou égale à environ 2 % en poids, les pourcentages étant calculés par rapport au poids total du matériau, et présentant une absorbance dans le spectre visible ; et dont l'absorption de la lumière à 500 nm est supérieure à 50 %, de préférence supérieure à 60 %, de l'absorption de la lumière à 400 nm.

Un tel matériau a pour avantage d'être activable grâce à l'apport d'énergie solaire. Ainsi, l'apport d'une source de rayonnement UV artificiel n'est plus nécessaire ; une exposition aux rayonnements solaires suffit. L'activation de ce matériau est donc économique et présente peu d'inconvénients environnementaux.

Le TiO2 de ce matériau peut être présent sous forme d'un monolithe, en particulier d'un film monolithique.

Il peut être en particulier obtenu par le procédé ci-dessus.

Il est aussi proposé l'utilisation du matériau décrit ci-dessus en tant que photocatalyseur pour la dégradation de polluants dans l'air ou dans l'eau sous rayonnement dans le spectre visible. Une autre utilisation d'un tel matériau en tant que photocatalyseur est le craquage de l'eau en H₂ et O₂.

Ainsi, la dépollution dans l'air ou dans l'eau est effectuée avec comme apport énergétique unique ou au moins principale l'énergie solaire. Le même avantage existe pour le craquage de l'eau en H₂ et O₂.

### Dessins

D'autres objectifs, caractéristiques et avantages ressortiront de la description donnée ci-après à titre illustratif et non limitatif, en référence aux dessins parmi lesquels :
- la figure 1 est un diagramme illustrant les étapes du procédé pour la production d'un matériau à base de TiO₂ selon l'invention ;
- la figure 2 est un graphe montrant les mesures DRX pour les matériaux des exemples 1 à 4, ainsi que pour le TiO₂ de référence (anatase, Aldrich) en unité arbitraire en fonction de l'angle 2θ pour des valeurs de celui-ci comprises entre 5° et 70° ;
- la figure 3 est un graphe montrant l'absorbance des matériaux des exemples 1 à 5 et celle du TiO₂ de référence (anatase), en unité Kubelka-Munk en fonction de la longueur d'onde pour des valeurs de celle-ci comprises entre 200 nm et 700 nm ;
- la figure 4 est un graphe montrant l'absorbance de solutions contenant un colorant orange G dans lesquelles les matériaux des exemples 2 à 4 et du TiO₂ de référence (anatase, Aldrich) ont été ajoutés, après exposition à la lumière pendant 1h30, en fonction de la longueur d'onde pour des valeurs de celle-ci comprises entre 250 nm et 700 nm ;
- la figure 5 est un graphe représentant l'absorbance des solutions contenant un colorant orange II dans lesquelles le matériau de l'exemple 3 a été ajouté, après exposition au rayonnement du soleil et dans l'obscurité, en fonction du temps entre 0 et 500 min; et
- la figure 6 est un graphe représentant l'absorbance des solutions contenant un colorant orange II dans lesquelles du TiO₂ Degussa P25 a été ajouté, après exposition au rayonnement du soleil et dans l'obscurité, en fonction du temps entre 0 et 500 min.

### Description

### Procédé

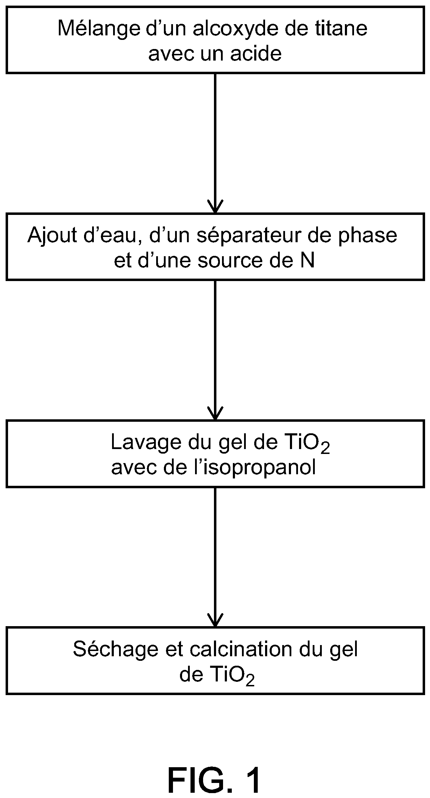

En référence à la figure 1 est décrit ci-après un procédé pour la fabrication d'un matériau à base de TiO₂ présentant une mésoporosité et/ou une macroporosité et comprenant au moins 60 %, de préférence au moins 70 %, en poids de TiO₂ sous forme d'anatase et du carbone élémentaire (C) et/ou de l'azote élémentaire (N) sous forme de trace, les pourcentages étant calculés par rapport au poids total du matériau.

La quantité de C et N élémentaires est déterminée par analyse chimique selon la méthode décrite ci-dessous dans la partie « Méthodes ».

L'anatase désigne dans l'ensemble du présent exposé un oxyde de titane (TiO₂) tétragonal, à groupe d'espace I4₁/amd, ayant pour paramètre de maille : a = 3,7852 Å (angström) ; c = 9,5139 Å, et de densité théorique 3,893. Ces chiffres peuvent légèrement varier de ± 0,05 Å (pour a et c) et de ± 0,1 (pour la densité).

Le procédé comprend :
- le mélange préalable d'un alcoxyde de titane avec un acide ;
- l'ajout d'eau, d'un séparateur de phase et d'une source de N au mélange préalable pour obtenir un gel de TiO₂ ;
- le lavage du gel de TiO₂ avec de l'isopropanol (iPrOH) ;
- le séchage et la calcination du gel de TiO₂ afin de produire le matériau à base de TiO₂ exempt de métaux et présentant des mésopores et macropores ouvertes, les mésopores présentant un diamètre compris entre 2 nm et 10 nm, les macropores présentant un diamètre compris entre 2 µm et 10 µm, le volume mésoporeux étant supérieur à 0,1 cm³/g et le volume macroporeux étant supérieur à 0,3 cm³/g, le matériau à base de TiO² comprenant au moins 60 %, de préférence au moins 70 %, en poids de TiO₂ sous forme d'anatase et du carbone élémentaire et/ou de l'azote élémentaire, le carbone élémentaire et/ou l'azote élémentaire étant chacun en une quantité inférieure ou égale à environ 2 % en poids, les pourcentages étant calculés par rapport au poids total du matériau.

Le mélange préalable de l'alcoxyde de titane avec un acide signifie que ces deux composés sont mis en présence l'un de l'autre avant de les mélanger avec d'autres composés utilisés pour le procédé.

Le mélange préalable peut être réalisé dans un bain de glace à environ 0°C et sous agitation. L'agitation du mélange est avantageusement progressive : l'alcoxyde de titane est tout d'abord placé à 0°C sous agitation lente, notamment pour s'assurer que le barreau aimanté ne se bloque pas dans l'alcoxyde de titane. Au fur et à mesure que l'acide est ajouté à l'alcoxyde de titane, le mélange devient fumant et moins visqueux, la vitesse d'agitation est alors augmentée jusqu'à une valeur comprise entre environ 300 tr/min et environ 400 tr/min, de préférence entre environ 300 tr/min et environ 350 tr/min. Au-delà de 400 tr/min, il y a un risque pour que le barreau aimanté se bloque dans le mélange.

L'alcoxyde de titane désigne un composé de formule générale Ti(OR₁)(OR₂)(OR₃)(OR₄) où chacun des R₁ à R₄ désigne, indépendamment des autres, une chaine carbonée ramifiée comprenant entre 3 et 8 atomes de carbone, de préférence entre 3 et 5 atomes de carbone.

Les chaînes carbonées ramifiées comprenant entre 3 et 5 atomes de carbones sont choisies parmi le groupe constitué de : -CH(CH₃)₂ (isopropyle) ; - CH(CH₃)-CH₂-CH₃ (*sec*-butyle) ;-CH₂-CH(CH₃)₂ (isobutyle) ;-CH(CH₃)₃ (*tert-*butyle) ; -CH(CH₃)-CH₂-CH₂-CH₃ (1-méthylbutyle); -CH(CH₃)-CH(CH₃)₂ (1,2-diméthylpropyle) ; -CH₂-CH(CH₃)-CH₂-CH₃ (2-méthylbutyle) ; -CH₂-C(CH₃)₃ (2,2-diméthylpropyle) ; -(CH₂)₂-CH(CH₃)₂ (3-méthylbutyle) ; -CH(CH₂-CH₃)₂ (1-éthylpropyle) ; et -C(CH₃)₂-CH₂-CH₃ (1,1-diméthylpropyle).

Avantageusement, R₁ à R₄ désignent la même chaine carbonée comprenant entre 3 et 5 atomes de carbone. Ainsi, l'alcoxyde de titane peut être choisi parmi le groupe constitué de : Ti(OiPr)₄ (tétra(isopropoxyde) de titane) ; Ti(O*sec*-Bu)₄ (tétra(*sec*-butoxide) de titane) ; Ti(OiBu)₄(tétra(isobutoxyde) de titane) ; Ti(O*tert*-Bu)₄(tétra(*tert*-butoxide) de titane) ; Ti(OCH(CH₃)-CH₂-CH₂-CH₃)₄ (tétra(1-méthylbutanolate) de titane) ; Ti(OCH(CH₃)-CH(CH₃)₂)₄ (tétra(1,2-méthylpropanolate) de titane) ; Ti(OCH₂-CH(CH₃)-CH₂-CH₃)₄ (tétra(2-méthylbutanolate) de titane) ; Ti(OCH₂-C(CH₃)₃)₄ (tétra(2-diméthylpropanolate) de titane) ; Ti(O(CH₂)₂-CH(CH₃)₂)₄ (tétra(3-méthylbutanolate) de titane) ; Ti(OCH(CH₂-CH₃)₂)₄ (tétra(1-éthylpropanolate) de titane) ; et Ti(OC(CH₃)₂-CH₂-CH₃)₄ (tétra(1-diméthylpropanolate) de titane).

De préférence, la chaine carbonée ramifiée est le radical iPr (isopropyle : -CH(CH₃)₂). Ainsi, l'alcoxyde de titane présente de préférence la formule : Ti(OiPr)₄.

L'acide désigne un composé donneur de proton. Le ratio molaire alcoxyde de titane:acide est avantageusement compris entre 1:1 et 1:0,5. L'acide peut être choisi parmi le groupe constitué de : HCl (acide chlorhydrique), HNO₃ (acide nitrique), H₂SO₄ (acide sulfurique) et CH₃COOH (acide acétique).

De préférence, l'acide est HCl. Dans ce dernier cas, et si l'alcoxyde de titane est le Ti(OiPr)₄, le ratio molaire Ti(OiPr)₄:HCl est avantageusement compris entre 1:1 et 1:0,5.

Le séparateur de phase désigne un composé s'intercalant, à mesure que le TiO₂ se forme, entre ce dernier et l'eau, ce qui permet d'obtenir une structure macroporeuse après son élimination. De manière générale, le séparateur est un polymère PEO ou un copolymère comprenant au moins un bloc PEO (poly(oxyde d'éthylène)). Parmi les polymères PEO, les PEO de masse moyenne en nombre d'environ 10 000 et d'environ 20 000 sont préférés. Parmi les copolymères dibloc, on peut citer PEOnPPOm (copolymère comprenant un premier bloc avec n unités d'oxyde d'éthylène et un second bloc avec m unités d'oxyde de propylène). Parmi les copolymères tribloc, on peut citer PeOnPPOmPEOn (copolymère comprenant un premier bloc avec n unités d'oxyde d'éthylène, un deuxième bloc avec m unités d'oxyde de propylène et un troisième bloc avec n unités d'oxyde d'éthylène).

Le ratio alcoxyde de titane:séparateur de phase:eau détermine l'homogénéité des macropores au sein du matériau à base de TiO₂. Avantageusement, le ratio molaire alcoxyde de titane:EO (unités monomère EO dans le séparateur) est compris entre 1:0,6 et 1:0,2. Le ratio molaire alcoxyde de titane: H₂O est compris entre 1:3 et 1:15.

Dans le cas où l'alcoxyde de titane est le Ti(OiPr)₄, le ratio molaire Ti(OiPr)₄:EO est de préférence compris entre 1:0,6 et 1:0,2. Le ratio molaire Ti(OiPr)₄:H₂O est de préférence compris entre 1:3 et 1:15.

La source de N (azote) est un composé susceptible de libérer au moins un atome de N. Avantageusement, le ratio molaire alcoxyde de titane:source de N est compris entre 1:1 et 1:0,75.

La source de N est de préférence l'urée, NH4NO3 (nitrate d'ammonium) ou NFA (N-méthylformamide). De préférence, la source de N est NFA. Dans ce dernier cas, et si l'alcoxyde de titane est le Ti(OiPr)₄, le ratio molaire Ti(OiPr)₄:NFA est compris entre 1:1 et 1:0,75.

Dans une mise en oeuvre particulière, un alcool peut être ajouté en même temps que le séparateur de phase, l'eau et la source de N. De préférence, cet alcool est du EtOH (éthanol). Dans ce cas, le ratio molaire Ti(OiPr)₄:EtOH est avantageusement compris entre 1:1,5 et 1:1.

La gélation du TiO₂ est obtenue par exemple après exposition à une température comprise entre environ 20°C et environ 80°C, de préférence entre environ 30°C et environ 60°C, de préférence encore à environ 40°C, jusqu'à gélation complète du TiO₂.

Après gélation complète, le gel de TiO₂ est laissé pour maturation à une température comprise entre environ 40°C et environ 80°C, de préférence entre environ 50°C et environ 70°C, de préférence encore à environ 60°C. Le gel de TiO₂ est maturé pendant une durée comprise entre environ 10 h et environ 40 h, de préférence entre 15 h et environ 30 h, encore de préférence environ 24 h.

La calcination du gel de TiO₂ séché est avantageusement effectuée entre 250°C et 600°C, de préférence entre 300°C et 500°C, plus préférentiellement encore entre 325°C et 375°C, pendant une durée comprise entre 2 h et 12 h, de préférence entre 4 h et 6 h, plus préférentiellement encore entre 4 h 30 et 5 h 30. Par exemple, la calcination du gel de TiO₂ est réalisée à environ 350°C pendant environ 5 heures, avec une montée en température à 0.5°C/min.

Après la calcination, le solide est refroidit à température ambiante.

De manière alternative, une deuxième calcination peut être effectuée après le refroidissement dans les mêmes conditions que la première calcination.

### Matériau

Un matériau à base de TiO₂ est décrit ci-après. Ce matériau peut être obtenu à partir du procédé décrit ci-dessus.

Ce matériau présente une mésoporosité et/ou une macroporosité et comprend au moins 60 %, de préférence au moins 70 %, en poids de TiO₂ sous forme d'anatase et du carbone élémentaire et de l'azote élémentaire sous forme de trace, les pourcentages étant calculés par rapport au poids total du matériau. Il présente une absorbance dans le spectre visible et, en particulier, l'absorption de la lumière à 500 nm est supérieure à 50 %, de préférence supérieure à 60 %, de l'absorption de la lumière à 400 nm.

Le « spectre visible », également appelé « spectre optique », désigne la partie du spectre électromagnétique qui est visible pour l'oeil humain et communément compris entre environ 400 nm et environ 800 nm. Il correspond à ce qu'on appelle aussi « lumière » qui désigne l'ensemble des ondes électromagnétiques visibles par l'oeil humain. Les termes de « spectre visible », « spectre optique » ou « lumière » sont pris dans l'ensemble du présent exposé comme étant des synonymes. L'utilisation d'un terme ou d'un autre dans le présent exposé sera donc comprise comme une simple variation de formulation verbale et non comme déterminant une quelconque distinction.

La quantité de trace de C élémentaire et/ou de N élémentaire est déterminée par analyse chimique selon la méthode décrite ci-dessous.

Le C élémentaire et le N élémentaire sont présents sous forme de trace, c'est-à-dire que la quantité de chacun est inférieure ou égale à environ 2 %, de préférence inférieure ou égale à environ 1,5 %, encore de préférence inférieure à égale à environ 1 %, en poids par rapport au poids total du matériau. Avantageusement, la quantité totale de N et de C est de préférence inférieure à 2,5 %.

Le TiO₂ est sous la forme d'objets présentant des dimensions au moins micrométrique, c'est-à-dire qu'au moins deux des dimensions de ces objets sont égales ou supérieures à 1 µm. Ainsi, ce TiO₂ est plus facile à manipuler par rapport aux nanoparticules de TiO₂ de l'art antérieur et peut être aisément séparé de solutions dans lesquelles il a été ajouté, par exemple par sédimentation.

Lorsqu'aucune des dimensions des objets n'est supérieure ou égale à 5 mm, le TiO₂ est présent sous forme de poudre. La poudre peut être compactée en comprimés pour former des objets encore plus grands.

Lorsque ces objets présentent au moins une dimension supérieure ou égale à 5 mm, on parlera alors de monolithes. Le matériau sous forme de monolithes, notamment quand les trois dimensions sont supérieures à 3 mm, de préférence au moins une dimension supérieure à 1 cm, est encore plus facile à manipuler.

Les monolithes composant le matériau peuvent avoir une forme de bâtons, de cylindres creux, de plaques, etc. En particulier, lorsqu'ils ont une forme de cylindres creux ou de plaques, ils peuvent être présents seuls ou fixés à un support.

Qu'il soit sous forme de poudre ou de monolithes, le matériau présente de préférence des macropores et mésopores ouverts. Le volume mésoporeux du matériau est supérieur à environ 0,1 cm³/g, de préférence supérieur à environ 0,2 cm³/g, plus préférentiellement encore supérieur à environ 0,23 cm³/g. Il présente des mésopores de diamètre compris entre environ 2 nm et environ 10 nm, de préférence entre environ 3 nm et environ 8 nm, plus préférentiellement encore entre environ 4 nm et environ 5 nm.

Le volume macroporeux du matériau est supérieur à environ 0,3 cm³/g, de préférence supérieur à environ 0,5 cm³/g, plus préférentiellement encore supérieur à environ 0,7 cm³/g. Il présente des macropores de diamètre compris entre 2 µm et 10 µm, de préférence entre 3 µm et 8 µm, plus préférentiellement encore entre 4 µm et 5 µm.

De manière générale, la macroporosité du matériau permet de faire passer un fluide à travers celui-ci en exerçant un différentiel de pressions de faible valeur, c'est-à-dire inférieur à environ 10 bars, de préférence inférieur à environ 7 bars, plus préférentiellement encore entre environ 1 bar et environ 5 bars afin de pouvoir utiliser ce matériau pour des traitements catalytiques en continu.

Par ailleurs, ces caractéristiques de macroporosité et de mésoporosité permettent d'éviter une perte de charge lorsqu'un différentiel de pressions est appliqué.

Le matériau est exempt de métaux ce qui rend la synthèse plus simple car il n'y a besoin d'ajouter aucun additif métallique, supprimant ainsi la nécessité de prévoir la réduction de métaux avant leur utilisation. Ainsi, le matériau est plus économique.

Le matériau peut être utilisé en tant que photocatalyseur pour la dégradation de polluants dans l'air ou dans l'eau sous rayonnement dans le spectre visible.

Il peut encore être utilisé en tant que photocatalyseur pour le craquage de l'eau en H₂ (hydrogène) sous rayonnement dans le spectre visible.

### Méthodes

### Mesure DRX (diffraction aux rayons-X)

La mesure DRX des matériaux est réalisée avec un appareil Bruker D8 Advance en utilisant la raie Kα du cuivre avec λ = 1,5405 Å, 2 theta de 4° à 70°, par pas de 0.02°.

### Mesure des spectres d'absorption

Les spectres de réflexion diffuse (DRUV) des échantillons solides sont mesurés avec un spectrophotomètre PerkinElmer Lambda-35 équipé d'une sphère intégrante (Labsphère) en utilisant comme référence BaSO₄. Les spectres DRUV sont ensuite convertis en unités Kubelka-Munk (F(R)) pour obtenir les spectres d'absorption.

### Analyse chimique pour la détermination de la quantité de N, de C et de H

Les analyse des éléments (N,C,H) ont été réalisées par combustion sur un appareil Fisons EA1108.

### Test de dégradation du colorant orange G

Dans un réacteur (en quartz ou en verre) de 50 mL, 20 mL de solution aqueuse de colorant orange G y est placé. La solution est acidifiée avec HCl jusqu'un pH inférieur à 5. Le colorant orange G est introduit dans le réacteur dans une quantité suffisante pour atteindre la concentration de 80 mg/L. 150 mg du matériau analysé sont introduits dans le réacteur (sauf pour l'échantillon de contrôle).

Le réacteur est ensuite placé sous une lampe au Ne (néon) afin d'éclairer le matériau sous lumière visible pendant 1 h 30. Certains des matériaux peuvent être disposés dans l'obscurité pendant le même temps pour comparaison.

Le surnageant est analysé par spectrométrie UV-visible dans une cuve de 1 cm de longueur (chemin optique) avec un spectrophotomètre Perkin-Elmer Lambda-40 (double faisceau), avec comme référence une solution aqueuse de HCl. Les résultats sont analysés dans une gamme de longueur d'onde comprise entre 250 et 700 nm.

### Test de dégradation du colorant orange 7 sous rayonnement solaire

Une solution aqueuse de 50 mL présentant une concentration de 10⁻⁴ mol/L de colorant acide orange 7 (aussi connu sous le nom de colorant orange II : obtenu chez Sigma Aldrich sous la dénomination AO7 sodium salt, BioXtra, Dye content > 85 %, 10-4M) est préparée et acidifiée à pH 3.

12,5 mg de matériau sont placés dans la solution aqueuse et le tout est exposé au rayonnement solaire ou conservé dans l'obscurité sous agitation. Des prélèvements réguliers de 4 mL de la solution sont effectués et centrifugés à 5 000 tr/min pendant 10 min.

Le surnageant est analysé par spectrométrie UV-visible dans une cuve de 1 cm de longueur (chemin optique) avec un spectrophotomètre Shimadzu UV-2450, avec comme référence une solution aqueuse de HCl. Les résultats sont analysés à une longueur d'onde de 484 nm correspondant au maximum d'absorption du colorant orange II.

### Exemples

Le tableau 1 ci-dessous présente les quantités des produits, exprimées en ratios molaires par rapport à la quantité d'alcoxyde de titane. « x » signifie alors x moles pour 1 mole d'alcoxyde de titane.

**Tableau 1**

| | Exemple 1 | Exemple 2 | Exemple 3 | Exemple 4 |
|---|---|---|---|---|
| Ti(OiPr)₄ | 1 | 1 | 1 | 1 |
| PEO | 0,57 | 0,56 | 0,56 | 0,57 |
| H₂O | 10,05 | 7,58 | 7,66 | 6,90 |
| HCl | 0,65 | 0,89 | 0,91 | 0,64 |
| NFA | 0,933 | 0,905 | 0,906 | 0,933 |
| EtOH | 0 | 0 | 0 | 1,23 |

Le Ti(OiPr)₄ est au préalable mélangé à du HCl pour son acidification dans un réacteur. L'eau, le PEO (Mn = 10 000 sauf pour l'exemple 3 où Mn = 20 000), le NFA (N-méthylformamide) et éventuellement l'EtOH (éthanol) sont disposés sur glace avant d'être ajoutés au mélange. L'ensemble est mélangé alors que le réacteur est plongé dans un bain de glace pendant 4 min. Le réacteur est ensuite fermé hermétiquement et exposé à 40°C pour la gélation. Après gélation complète, le gel de TiO₂ obtenu est laissé à 60°C pour maturation pendant 24 heures. Le gel de TiO₂ est ensuite lavé avec de l'iPrOH (isopropanol) et séché par évaporation à 40°C pendant une semaine. Le gel de TiO₂ séché est prétraité en étuve à 40°C et après calciné à 350°C pendant 5 heures.

Un cinquième exemple correspondant à l'exemple 2 mais ayant subi une deuxième calcination dans les mêmes conditions que la première calcination a été testé.

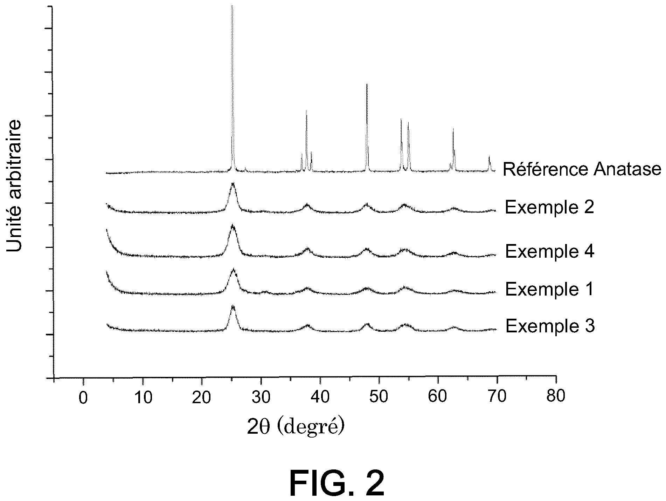

La figure 2 montre les mesures DRX pour les exemples 1 à 4 par rapport à un anatase de référence qui est le TiO₂ (anatase, Aldrich) (vendu sous la référence 248576, -325 mesh, pureté > 99 %, par Sigma-Aldrich). On observe donc que le matériau obtenu est bien de l'anatase pour ces exemples.

Les exemples 1, 2 et 5 donnent des matériaux sous forme de monolithes et de poudre, l'exemple 3 donne un matériau sous forme de poudre et l'exemple 4 sous forme de monolithes.

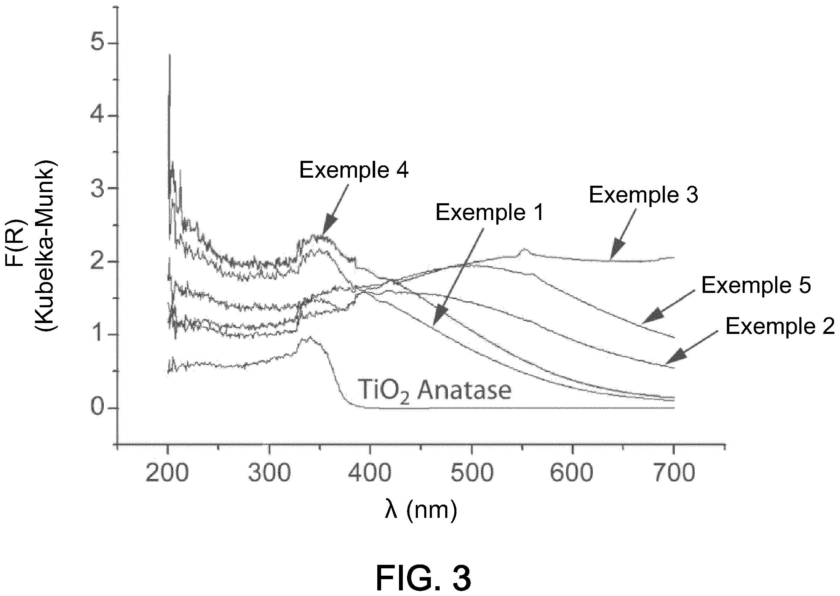

L'absorbance F(R) (Kubelka-Munk) des exemples 1 à 5 est mesurée entre 200 nm et 700 nm et est représentée sur la figure 3. On constate des courbes représentées sur la figure 3 que les matériaux des exemples 1 à 5 absorbent dans les longueurs d'onde supérieures à 400 nm contrairement au matériau anatase-Aldrich. Les ratios (A₅₀₀ₙₘ/A₄₀₀ₙₘ) entre l'absorbance à 500 nm et à 400 nm des matériaux des exemples 1 à 5 sont donnés dans le tableau 2 suivant.

**Tableau 2**

| | Exemple 1 | Exemple 2 | Exemple 3 | Exemple 4 | Exemple 5 |
|---|---|---|---|---|---|
| A₅₀₀ₙₘ/A₄₀₀ₙₘ | 51,5 % | 91,3% | 119 % | 56,7 % | 120% |

Le test de dégradation du colorant orange G a été réalisé pour trois des matériaux. Pour l'exemple 4, le test a été réalisé sous lampe et dans l'obscurité, ce qui permet de conclure que la dégradation du colorant orange G est bien due à la présente du matériau.

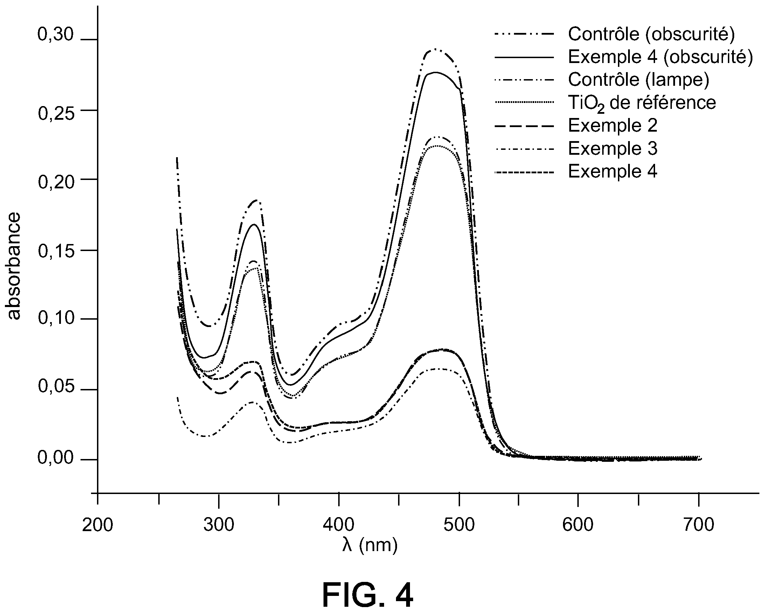

La figure 4 montre l'absorbance de la solution après exposition à la lumière pendant 1 h 30. Le tableau 3 présente les ratios (A/A_{ref}) entre l'absorbance à 500 nm des solutions ayant contenu les matériaux des exemples 2 à 4 et l'absorbance à 500 nm de l'échantillon de contrôle (c'est-à-dire sans photocatalyseur) exposé à la lumière.

**Tableau 3**

| | TiO₂ (anatase, Aldrich) | Exemple 2 | Exemple 3 | Exemple 4 |
|---|---|---|---|---|
| A/A_{ref} | 0,98 | 0,34 | 0,28 | 0,34 |

Ainsi, on observe que les matériaux des exemples 2 à 4 ont bien permis la dégradation du colorant orange G alors que TiO₂ (anatase, Aldrich) ne le permet pas.

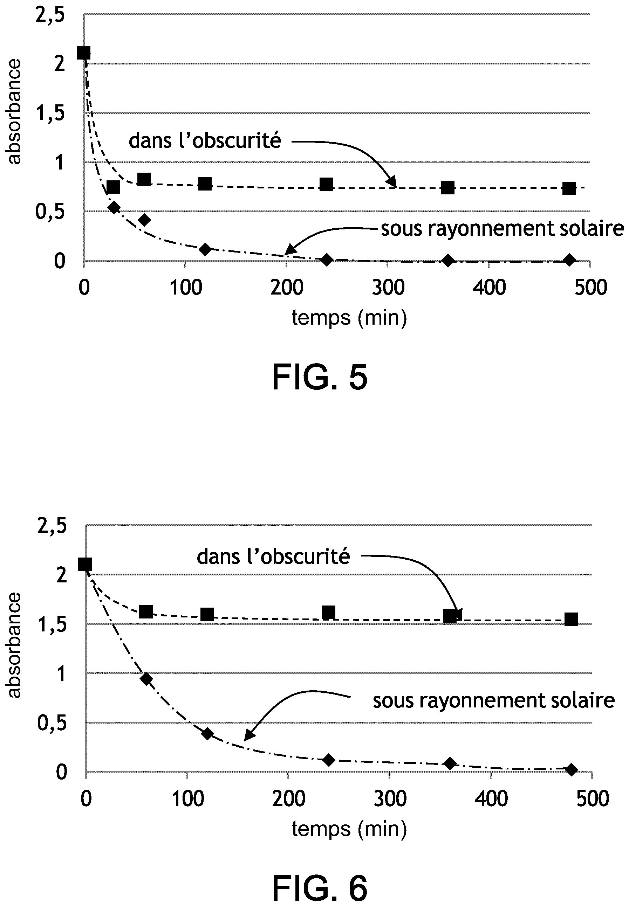

Le test de dégradation du colorant orange II a été réalisé pour le matériau de l'exemple 3 et le TiO₂ Degussa P25 (respectivement figures 5 et 6) sous deux conditions d'éclairage différentes : sous rayonnement solaire et dans l'obscurité. L'évolution de la solution contenant le matériau a été suivie pendant environ 8 heures (voir figure 5).

On peut constater à partir du graphe de la figure 5 que la solution ayant contenu le matériau de l'exemple 3 est complètement transparente après exposition à la lumière du soleil de 250 min contrairement à la solution ayant contenu le même matériau après disposition dans l'obscurité.

Par ailleurs, on remarque à partir de la figure 6 que bien que la solution ayant contenu le TiO₂ Degussa P25 devient complètement transparente à la fin du test soit après une exposition à la lumière du soleil supérieure à 500 min, ce résultat est obtenu plus tardivement que pour le matériau de l'exemple 3.

## Revendications

1. Procédé de production d'un matériau à base de TiO₂ exempt de métaux ainsi que d'additifs autres que C et N, le procédé comprenant :
- le mélange préalable d'un alcoxyde de titane avec un acide ;
- l'ajout d'eau, d'un séparateur de phase et d'une source de N au mélange d'alcoxyde de titane et d'acide pour obtenir un gel de TiO₂ ;
- le lavage du gel de TiO₂ avec de l'isopropanol (iPrOH) ;
- le séchage et la calcination du gel de TiO₂ afin de produire le matériau à base de TiO₂ exempt de métaux et présentant des mésopores et macropores ouvertes, les mésopores présentant un diamètre compris entre 2 nm et 10 nm, les macropores présentant un diamètre compris entre 2 µm et 10 µm, le volume mésoporeux étant supérieur à 0,1 cm³/g et le volume macroporeux étant supérieur à 0,3 cm³/g, le matériau à base de TiO₂ comprenant au moins 60 %, de préférence au moins 70 %, en poids de TiO₂ sous forme d'anatase et du carbone élémentaire et/ou de l'azote élémentaire, le carbone élémentaire et/ou l'azote élémentaire étant chacun en une quantité inférieure ou égale à environ 2 % en poids, les pourcentages étant calculés par rapport au poids total du matériau.

2. Procédé selon la revendication 1, dans lequel l'alcoxyde de titane est Ti(OiPr)₄.

3. Procédé selon la revendication 1 ou la revendication 2, dans lequel le ratio molaire alcoxyde de titane:acide est compris entre 1:1 et 1:0,5.

4. Procédé selon l'une des revendications 1 à 3, dans lequel l'acide est du HCl.

5. Procédé selon l'une des revendications 1 à 4, dans lequel le séparateur de phase est du poly(oxyde d'éthylène) (PEO), de préférence à une masse molaire moyenne en nombre (Mn) supérieure ou égale à 10 000.

6. Procédé selon l'une des revendications 1 à 5, dans lequel le ratio molaire alcoxyde de titane:source de N est compris entre 1:1 et 1:0,75.

7. Procédé selon l'une des revendications 1 à 6, dans lequel la source de N est du N-méthyleformamide (NFA).

8. Matériau à base de TiO₂ et exempt de métaux ainsi que des additifs autres que C et N, le matériau présentant des mésopores et macropores ouvertes, les mésopores présentant un diamètre compris entre 2 nm et 10 nm, les macropores présentant un diamètre compris entre 2 µm et 10 µm, le volume mésoporeux étant supérieur à 0,1 cm³/g et le volume macroporeux étant supérieur à 0,3 cm³/g, le matériau à base de TiO₂ comprenant au moins 60 %, de préférence au moins 70 %, en poids de TiO₂ sous forme d'anatase et du carbone élémentaire et/ou de l'azote élémentaire, le carbone élémentaire et/ou l'azote élémentaire étant chacun en une quantité inférieure ou égale à environ 2 % en poids, les pourcentages étant calculés par rapport au poids total du matériau,
et présentant une absorbance dans le spectre visible ; et
dont l'absorption de la lumière à 500 nm est supérieure à 50 %, de préférence supérieure à 60 %, de l'absorption de la lumière à 400 nm.

9. Matériau selon la revendication 8, dans lequel le TiO₂ est présent sous forme d'un monolithe.

10. Matériau selon la revendication 9, dans lequel le TiO₂ est présent sous forme d'un film monolithique.

11. Utilisation du matériau selon l'une des revendications 8 à 10 en tant que photocatalyseur pour la dégradation de polluants dans l'air ou dans l'eau sous rayonnement dans le spectre visible.

12. Utilisation du matériau selon l'une des revendications 8 à 10 en tant que photocatalyseur pour le craquage de l'eau en H₂ sous rayonnement dans le spectre visible.

## Patentansprüche

1. Verfahren zur Herstellung eines Materials auf TiO₂-Basis, welches frei von Metall sowie von anderen Additiven als C und N ist, wobei das Verfahren umfasst:
- das vorherige Mischen eines Titanalkoxids mit einer Säure;
- Zugeben von Wasser, einem Phasentrenner und einer N-Quelle zu der Mischung aus Titanalkoxid und Säure, um ein TiO₂-Gel zu erhalten;
- Waschen des TiO₂-Gels mit Isopropanol (iPrOH);
- Trocknen und Kalzinieren des TiO₂-Gels zur Herstellung des metallfreien Materials auf TiO₂-Basis, welches offene Mesoporen und Makroporen umfasst, wobei die Mesoporen einen Durchmesser zwischen 2 nm und 10 nm aufweisen, wobei die Makroporen einen Durchmesser zwischen 2 µm und 10 µm aufweisen, wobei das mesoporöse Volumen größer als 0,1 cm³/g und das makroporöse Volumen größer als 0,3 cm³/g ist, wobei das TiO₂-Material wenigstens 60 Gew.- %, vorzugsweise mindestens 70 Gew.-% TiO₂ in Form von Anatase und elementarem Kohlenstoff und/oder elementarem Stickstoff umfasst, wobei der elementare Kohlenstoff und/oder der elementare Stickstoff jeweils in einer Menge kleiner gleich ungefähr 2 Gew.-% vorliegen, wobei die Prozentsätze auf der Grundlage des Gesamtgewichts des Materials berechnet werden.

2. Verfahren nach Anspruch 1, wobei das Titanalkoxid Ti(OiPr)₄ ist.

3. Verfahren nach Anspruch 1 oder Anspruch 2, wobei das Molverhältnis Titanalkoxid:Säure zwischen 1:1 und 1:0,5 liegt.

4. Verfahren nach einem der Ansprüche 1 bis 3, wobei die Säure HCl ist.

5. Verfahren nach einem der Ansprüche 1 bis 4, wobei der Phasentrenner Polyethylenoxid (PEO) ist, vorzugsweise mit einem zahlenmittleren Molekulargewicht (Mn) größer oder gleich 10 000.

6. Verfahren nach einem der Ansprüche 1 bis 5, wobei das Molverhältnis Titanalkoxid:N-Quelle zwischen 1:1 und 1:0,75 liegt.

7. Verfahren nach einem der Ansprüche 1 bis 6, wobei die N-Quelle N-Methylformamid (NFA) ist.

8. Material auf TiO₂-Basis, welches frei von Metall sowie von anderen Additiven als C und N ist, wobei das Material offene Mesoporen und Makroporen aufweist, wobei die Mesoporen einen Durchmesser zwischen 2 nm und 10 nm aufweisen, wobei die Makroporen einen Durchmesser zwischen 2 µm und 10 µm aufweisen, wobei das mesoporöse Volumen größer als 0,1 cm³/g und das makroporöse Volumen größer als 0,3 cm³/g ist, wobei das TiO₂-Material wenigstens 60 Gew.-%, vorzugsweise mindestens 70 Gew.-% TiO₂ in Form von Anatase und elementarem Kohlenstoff und/oder elementarem Stickstoff umfasst, wobei der elementare Kohlenstoff und/oder der elementare Stickstoff jeweils in einer Menge kleiner gleich ungefähr 2 Gew.-% vorliegen, wobei die Prozentsätze auf der Grundlage des Gesamtgewichts des Materials berechnet werden,
und eine Absorption im sichtbaren Bereich aufweisen; und
wobei die Lichtabsorption bei 500 nm größer als 50 %, vorzugsweise größer als 60 % der Lichtabsorption bei 400 nm ist.

9. Material nach Anspruch 8, wobei TiO₂ als ein Monolith vorliegt.

10. Material nach Anspruch 9, wobei TiO₂ in Form eines monolithischen Films vorliegt.

11. Verwendung des Materials nach einem der Ansprüche 8 bis 10 als Photokatalysator für den Abbau von Schadstoffen in Luft oder Wasser unter Strahlung im sichtbaren Spektrum.

12. Verwendung des Materials nach einem der Ansprüche 8 bis 10 als Photokatalysator zum Spalten von Wasser in H₂ unter Strahlung im sichtbaren Spektrum.

## Claims

1. Method for producing a TiO₂-based material with no metals as well as additives other than C and N, the method comprising:
- the prior mixing of a titanium alkoxide with an acid;
- the adding of water, a phase separator and a source of N to the mixture of titanium alkoxide and acid to obtain a TiO₂ gel;
- the washing of the TiO₂ gel with isopropanol (iPrOH);
- the drying and the calcinating of the TiO₂ gel in order to produce the TiO₂-based material with no metals and having open mesopores and macropores, the mesopores having a diameter of between 2nm and 10nm, the macropores having a diameter of between 2µm and 10µm, the mesoporous volume being greater than 0.1cm³/g and the macroporous volume being greater than 0.3cm³/g, the TiO₂-based material comprising at least 60%, preferably at least 70%, by weight of TiO₂ in the form of anatase and elementary carbon and/or elementary nitrogen, the elementary carbon and/or the elementary nitrogen each being of a quantity less than or equal to around 2% by weight, the percentages being calculated with respect to the total weight of the material.

2. Method according to claim 1, wherein the titanium alkoxide is Ti(OiPr)₄.

3. Method according to claim 1 or claim 2, wherein the titanium alkoxide:acid molar ratio is between 1:1 and 1:0.5.

4. Method according to one of claims 1 to 3, wherein the acid is HCl.

5. Method according to one of claims 1 to 4, wherein the phase separator is poly(ethylene oxide) (PEO), preferably with an average molar mass in number (Mn) greater than or equal to 10000.

6. Method according to one of claims 1 to 5, wherein the titanium alkoxide:source of N molar ratio is of between 1:1 and 1:0.75.

7. Method according to one of claims 1 to 6, wherein the source of N is N-methyl formamide (NFA).

8. TiO₂-based material and with no metals as well as additives other than C and N, the material having open mesopores and macropores, the mesopores having a diameter of between 2nm and 10nm, the macropores having a diameter of between 2µm and 10µm, the macropores having a diameter of between 2µm and 10µm, the mesoporous volume being greater than 0.1cm³/g and the macroporous volume being greater than 0.3cm³/g, the TiO₂-based material comprising at least 60%, preferably at least 70%, by weight of TiO₂ in the form of anatase and elementary carbon and/or elementary nitrogen, the elementary carbon and/or the elementary nitrogen each being in a quantity less than or equal to around 2% by weight, the percentages being calculated with respect to the total weight of the material,
and having an absorbance in the visible spectrum; and
of which the absorption of light at 500nm is greater than 50%, preferably greater than 60%, of the absorption of light at 400nm.

9. Material according to claim 8, wherein the TiO₂ is present in the form of a monolith.

10. Material according to claim 9, wherein the TiO₂ is present in the form of a monolithic film.

11. Use of the material according to one of claims 8 to 10 as a photocatalyst to degrade pollutants in air or in water under radiation in the visible spectrum.

12. Use of the material according to one of claims 8 to 10 as a photocatalyst for the cracking of water into H₂ under radiation in the visible spectrum.
